## Europäisches Patentamt

## European Patent Office

## Office européen des brevets

(11) Publication number: **0 202 384**

**A1**

## EUROPEAN PATENT APPLICATION

(21) Application number: **85309476.1**

(22) Date of filing: **24.12.85**

(51) Int. Cl.⁴: **A 61 M 1/00**
**A 61 M 7/00**

(30) Priority: **27.12.84 GB 8432650**
**01.08.85 GB 8519391**

(43) Date of publication of application:
**26.11.86 Bulletin 86/48**

(84) Designated Contracting States:
**AT BE CH DE FR GB IT LI LU NL SE**

(71) Applicant: **BARD LIMITED**
**Pennywell Industrial Estate**
**Sunderland SR4 9EW(GB)**

(72) Inventor: **Brazier, Gary Brian**
**10 Main Road Great Oakley**
**Harwich Essex CO12 5AZ(GB)**

(72) Inventor: **Harris, Christopher**
**31 Frietuna Road**
**Frinton on Sea Essex CO13 0QP(GB)**

(74) Representative: **Moon, Donald Keith et al,**
**BREWER & SON 5-9 Quality Court Chancery Lane**
**London WC2A 1HT(GB)**

(54) **Pump for medical use.**

(57) A pump, which may be suitable for use as a bladder evacuator which combines the ease of use of the popular "Ellik" device with an efficient filtering action so as to avoid tidal flow of tissue pieces is based on a horizontal barrel (10) with a squeezable circulator bulb (17) at one end and a nozzle (11) at the other. Immediately beneath the barrel (10) is a reservoir bottle (23) within which is a large open-topped hollow filter element (28). Flow of liquid through the device is unidirectional from the circulator (17) along the barrel (10) to the nozzle (11) and hence to the patient's bladder (30), returning via the nozzle (11) to the reservoir bottle (23) then inwardly through the walls of the filter element (28) and back up into the circulator bulb (17). Flap valves (14,27) or a flap valve (14) and a valve shuttle in the barrel (10) achieve the required unidirectional flow. By making the barrel (10) and bottle (23) transparent, the constitution of the liquid flow can be monitored. The bottle (23) and filter element (28) are readily accessible for removal from the barrel (10), e.g. for analysis of their contents or for sterilization.

*FIG. 1.*

## PUMP

This invention relates to a pump, which may be suitable for use as a bladder evacuator or for post-operative flushing of blocked catheters, comprising a nozzle for connection by a tube to a bodily or other cavity and a fluid circulator for imposing on fluid within the tube cyclic changes of pressure to cause the fluid to flow alternately and repeatedly into and out of the cavity thereby to flush the cavity, the circulator and the tube being connected by two different fluid flow paths, namely, an efflux path for flow of fluid away from the cavity into a reservoir of the device and thence to the circulator, and a flushing path for flow of liquid from the circulator to the cavity, each of which paths including a non-return valve whereby, in use of the device, fluid flow is uni-directional, from the circulator through the flushing path to the cavity, and then from the cavity through the efflux path back to the circulator.

Such a pump is useful as a bladder evacuator to generate a fluid flow which serves to carry pieces of bodily tissue from the bladder to the reservoir. It is important that the return flow of fluid from the reservoir to the bladder cavity should not carry back as many tissue pieces as have been swept out of the bladder. Some means of concentrating the tissue pieces in the reservoir is required.

The usual circulator employed with bladder evacuators is a rubber bulb, alternately squeezed and released by the surgeon. Any tissue filter introduced into the flushing path for the purpose of confining tissue pieces to the reservoir should restrict the flow of liquid as little as possible so that the frequency of squeezing and release of the bulb can be as high as the surgeon desires. Further, the surface of the filter should not be such as to become blocked by pieces of tissue.

So far as the present applicants are aware, all attempts previous to the present proposal to meet the above requirements have failed to secure wide acceptance by users. A simple bladder evacuator known by the name "Ellik" continues to be the instrument of choice.

The Ellik device has a shape which resembles that of an hour-glass of which the upper vessel is connected to a rubber bulb and the lower is a collection bottle. Alternate squeezing and release of the bulb, when the device is full of liquid, induces outward and return flow of this liquid along a tube connected to the upper vessel of the device and to the bladder of a patient. Debris and other matter entrained in the return flow is collected in the bottle.

It is a problem with the Ellik device that it fails to trap all of the entrained matter in the collection bottle. Instead, some of it remains entrained in the liquid flow and is carried back to the bladder. Some at least of the entrained matter is seen to be "tidal", that is to say, carried backwards and forwards between the bladder and the collection bottle.

Proposals for improvement on the performance of the Ellik device are made in U.S. Patents Nos. 3892226 and 4282873 published 1975 and 1981 respectively.

U.S. Patent No. 3892226 to Rosen discloses a bladder evacuator which takes the form of a hand-grippable column divided longitudinally by a web into a downflow channel and an upflow channel. At the foot of the column is a reservoir, and at its head is a nozzle and a pumping bulb. In the web at opposite ends are two non-return flap valves, with the one at the foot of the column seating on an area in which is a plurality of small holes instead of one large one, the small holes serving to block the passage of tissue pieces more effectively than a single large hole.

It is one disadvantage of the Rosen device that the area of the seat of the flap valve at the foot of the column is insufficient for effective filtering. If the holes are usefully small they soon become blocked, but if they are made larger they allow small tissue pieces to pass through. Another disadvantage is that the tissue pieces which are caught on the valve seat are not easily cleaned off, are not included with the reservoir when it is removed from the column to enable its contents to be analysed, and may impede movement of the flap valve member and prevent full closure of the valve.

U.S. Patent No. 4282873 to Roth discloses an evacuator with a nozzle, a pumping bulb and a reservoir in which a quarter turn rotation of the bulb causes an integral stem to rotate within a manifold to close one aperture and open another. A combination of squeezing and releasing the bulb with forward and backward rotation of the bulb achieves the desired unidirectional flow including flow through a filter element carried on the manifold, but the co-ordinated hand movements required to operate the device are found in practice to hinder rapid pumping and deter users.

An earlier proposal is to be found in U.S. Patent No. 1925230 to Buckhout. The device offers the major advantages of a large filter area at a location within its reservoir, and non-return valves spaced from the filter, but it is not a convenient instrument to handle and use and therefore does not find favour with users.

A pumping device is disclosed in GB Patent Application No. 2136690A which is relatively convenient to handle, but it lacks an effective filter or unidirectional liquid flow path and so is not well-suited to achieve the sought-for improvement in filtering performance.

The problem which the present invention addresses is to provide a device which is as convenient to handle and use as the familiar Ellik instrument yet which offers a substantial improvement on its filtering performance.

A pump in accordance with the present invention is characterised by:

i)  a hollow barrel within which is a web dividing the barrel into a first chamber which communicates with the nozzle and a second chamber which communicates with the circulator, and an aperture in the web, controlled by the non-return valve of the flushing path, to allow liquid to pass through the aperture only from the second chamber to the first chamber;

ii) a reservoir bottle which is detachably secured at its neck to a bottle seating on the cylindrical surface of the barrel, the seating surrounding a first aperture in the cylindrical surface for communication between the first chamber and the bottle and a second aperture in the cylindrical surface for communication between the second chamber and the bottle;

iii) a hollow filter element with one open end for fitting onto a filter seating, which seating surrounds the said second aperture and lies within the bottle seating alongside the first aperture, the filter element extending away from the second aperture towards the base of the bottle over a substantial part of the length of the bottle and serving to prevent particulate matter in the efflux flow from flowing further than the reservoir bottle; and in that

iv) the non-return valve of the efflux path is located between the filter element and the fluid circulator and spaced from the filter element, the valve having a valve member which is movable between an open disposition to allow liquid to pass through the filter from the bottle to the circulator and a closed disposition to prevent flow in the opposite direction.

The use of a hollow filter element permits the specification of as large an area of filter surface as is deemed necessary. Filtering is performed on a flow of liquid which is inward through the walls of the filter element, so the arrangement must prevent inward collapse of the element during filtering. In one embodiment, the filter element is supported from

- 6 -

interchanged for different tasks. The filter and
bottle are readily detached together from the barrel
and so can be handled as a unit to be either discarded
or saved for analysis of their contents. When the
bottle and filter are removed, the remaining barrel and
circulator are easy to sterilize for re-use.

The non-return valves of the pump can be ordinary flap
valves. The non-return valves are both spaced
"downstream" of the filter element and so are subject
to a flow of filtered liquid devoid of tissue pieces.
Their operation should therefore be reliable.

For a better understanding of the present invention,
and to show more clearly how the same may be carried
into effect, reference will now be made, by way of
example, to the accompanying drawings in which:

Figure 1 is a transverse diametral section of a bladder
evacuator, being a first embodiment of the pump of the
present invention;

Figure 2 is a similar section, showing another point in
the pumping cycle of the device; and

Figures 3 and 4 are similar, but fragmentary, sections
of a second embodiment of the pump.

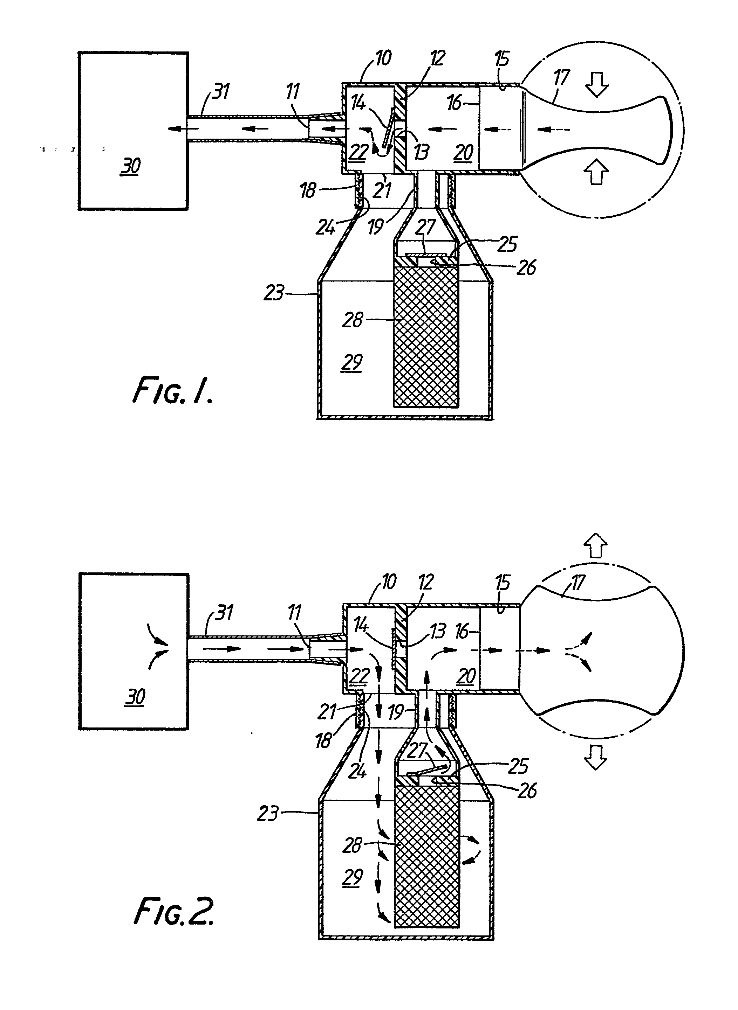

The evacuator shown in the Figures 1 and 2 comprises a
plastics barrel 10, formed with a spigot nozzle 11, an
internal web 12 with an aperture 13 covered by a
non-return flap valve 14, and an open end 15 in which
is fitted the neck 16 of a squeezable circulator bulb 17. The barrel has a large diameter threaded bottle seating 18 on its lower side and, within the diameter of the bottle seating 18, a first aperture 21 communicating with a first chamber 22 within the barrel and a second aperture formed by a dip tube 19 which communicates with a second chamber 20 within the barrel, the chambers 20 and 22 being separated by the web 12. A reservoir bottle 23 has a threaded neck portion 24 which engages the bottle seating 18.

Within the dip tube 19 is a flap valve and filter element seating 25 which includes an aperture 26 covered on its upper surface by a flap valve 27. A hollow filter element in the form of a filter sock 28 is fitted at its open upper end to the filter seating 25. Although not visible, the dip tube extends below the seating 25 as an open-walled structure which supports the sock 28 against compression while affording as large an area as possible within the surface area of the filter sock 28 for flow of liquid from the chamber 29 defined by the reservoir bottle 23 into the interior of the dip tube 19. It can be seen that the flap valve 27 is spaced from the filtering medium of the filter sock 28.

In use, the device is filled with fluid and connected to medical equipment, such as a resectoscope tube 31 for evacuation of the bladder 30 or some other bodily cavity of a patient.

Compression of the bulb 17, as shown in Figure 1, drives liquid from the bulb 17 along a flushing path, that is, through the non-return valve 13,14, and down the tube 31 to the bladder 30. Release of the bulb 17 generates a reduced pressure within the bulb, and therefore across the web 12, so that liquid within the chamber 22 presses the flap 14 down on the periphery of the aperture 13. At the same time, a pressure difference is established across the web 25 within the dip tube 19, causing the flap 27 to open and liquid to be drawn from the reservoir chamber 29 through the material of the filter sock 28 and into the bulb 17. Simultaneously, liquid flows from the bladder 30 into the chamber 29, to replace the liquid being drawn up the dip tube 19.

In effect, liquid flows along an efflux path, from the bladder 30 to the bulb 17. Pieces of tissue from the bladder 30 are carried into the reservoir chamber 29 but are prevented from entering the dip tube 19 by the action of the filter sock 28.

Each time the bulb recovers to its full size it is squeezed again by the surgeon, for as many times as he considers necessary. The reservoir bottle 23 and indeed the barrel 10 and bulb 17 may be made of transparent material if desired, so that the contents of the device can be viewed whenever desired.

Because the filter sock 28 has a large surface area, it does not readily become blocked by tissue pieces nor does it impose any substantial resistance to flow of liquid through the device. It will be appreciated that, with removal of the reservoir chamber 23 from the barrel 10, removal of a used filter sock 28, and replacement with a fresh sock, is readily possible.

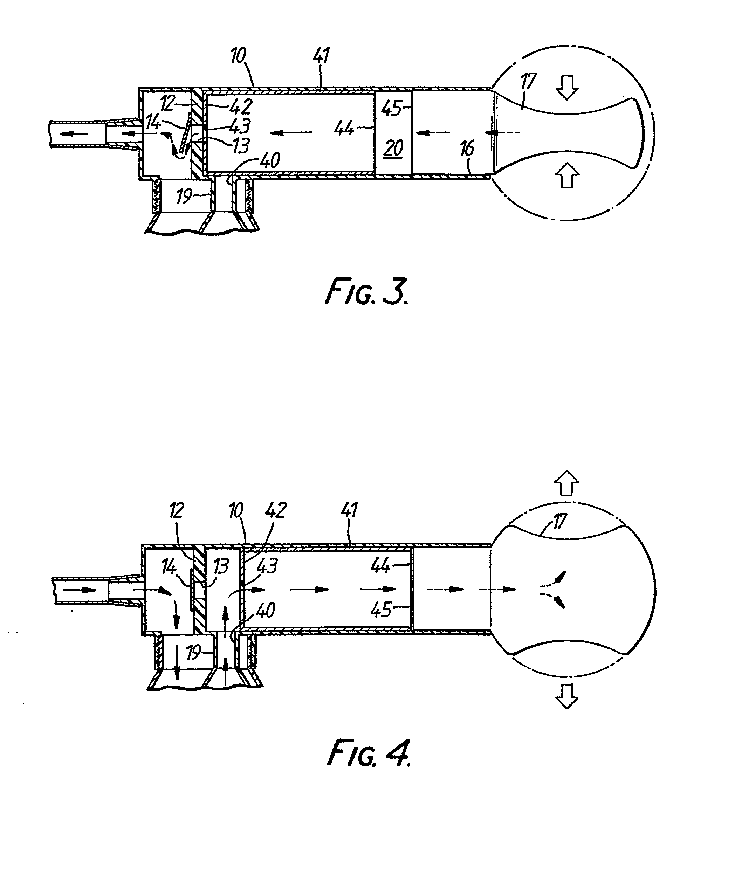

The embodiment of Figures 3 and 4 is similar, and the same reference numerals are used whenever the embodiment has the same construction. Instead of the apertured web 25 and flap valve 27, there is employed the aperture 40, formed in the cylindrical wall of the chamber 20 by the dip tube 19, and a shuttle valve member 41. The shuttle is accommodated in the barrel 10 for free sliding movement but with a liquid-tight or substantially liquid-tight fit. It has an open end 44 facing the bulb 17 and an end wall 42 facing the web 12 and including an aperture 43 which is aligned with the aperture 13.

In the flushing disposition of Figure 3, the shuttle, driven by the compression of the bulb 17 into abutment with the web 12, occludes the aperture 40, in a closed disposition of the shuttle 41, to prevent liquid flowing down the dip tube 19.

Upon release of the bulb 17, the flow of liquid back towards the bulb 17, as the flap 14 closes, urges the shuttle along the barrel towards the bulb 17. As soon as the shuttle starts to move in this way, the aperture 40 becomes increasingly exposed, allowing the flow into the bulb 17 to continue, but along the efflux path through the reservoir 29. The shuttle soon reaches the fully open efflux disposition of Figure 4 in which the open end 44 abuts the open end 45 of the neck 16 of the bulb 17.

In other embodiments the dip tube and filter sock can be replaced by an open-topped cylindrical or conical In other embodiments the dip tube and filter sock can be replaced by an open-topped cylindrical or conical filter element or filter cup which is self-supporting in compression and engages with a seating on the cylindrical surface of the barrel 10 within the bottle seating flange 18. With such a filter, a flap valve like that of Figure 1, a shuttle like Figure 3 or some other non-return valve, can all be used.

The illustrated pumps are also useful for flushing of blocked catheters, for example, after an operation, by connecting the spigot nozzle 11 to the available end of the catheter tube and alternatively squeezing and releasing the bulb 17 while the device is full of liquid.

0202384

- 11 -

<u>CLAIMS</u>

1.      A pump comprising a nozzle (11) for connection by a tube (31) to a bodily or other cavity (30) and a fluid circulator (17) for imposing on fluid within the tube cyclic changes of pressure to cause the fluid to flow alternately and repeatedly into and out of the cavity thereby to flush the cavity, the circulator and the tube being connected by two different fluid flow paths, namely, an efflux path for flow of fluid away from the cavity into a reservoir (29) of the device and thence to the circulator, and a flushing path for flow of liquid from the circulator to the cavity, each of which paths including a non-return valve (27,14) whereby, in use of the device, fluid flow is uni-directional, from the circulator through the flushing path to the cavity, and then from the cavity through the efflux path back to the circulator, the pump being characterised by:

i) a hollow barrel (10) within which is a web (12) dividing the barrel into a first chamber (22) which communicates with the nozzle (11) and a second chamber (20) which communicates with the circulator (17), and an aperture (13) in the web, controlled by the non-return valve (14) of the flushing path, to allow liquid to pass through the aperture only from the second chamber to the first chamber;

ii) a reservoir bottle (23) which is detachably secured at its neck (24) to a bottle seating (18) on the cylindrical surface of the barrel, the seating surrounding a first aperture (21) in the cylindrical surface for communication between the first chamber

0202384

- 12 -

(22) and the bottle and a second aperture (19) in the cylindrical surface for communication between the second chamber (20) and the bottle;

iii)  a hollow filter element (28) with one open end for fitting onto a filter seating (25), which seating surrounds the said second aperture (19) and lies within the bottle seating (18) alongside the first aperture (21), the filter element extending away from the second aperture towards the base of the bottle over a substantial part of the length of the bottle and serving to prevent particulate matter in the efflux flow flowwing further than the reservoir bottle;  and in that

iv)  the non-return valve (25,26,27) of the efflux path is located between the filter element (28) and the fluid circulator (17) and spaced from the filter element, the valve having a valve member (27) which is movable between an open disposition to allow liquid to pass through the filter from the bottle (23) to the circulator (17) and a closed disposition to prevent flow in the opposite direction.

2.      A pump as claimed in claim 1 characterised in that said filter seating is provided on a dip tube (19) on the barrel, at a position spaced from the cylindrical surface of the barrel (10).

3.      A pump as claimed in claim 2 characterised in that the non-return valve (25,26,27) of the efflux path is located within the length of the dip tube (19).

4.      A pump as claimed in claim 3 characterised in that the dip tube (19) has an apertured transverse web (25) which carries a non-return flap valve member (27) which co-acts with the aperture (26) in the transverse web, the web serving also as the filter seating.

5.      A pump as claimed in claim 1 or 2 characterised in that the flap valve of the efflux path comprises a shuttle valve member (41) accommodated within the barrel (10) for sliding movement within the second chamber (20) between a closed disposition in which it occludes said second aperture (19) and an open disposition nearer the circulator (17), in which it exposes the second aperture (19) to allow flow of liquid along the flushing path.

6.      A pump as claimed in claim 5 characterised in that the shuttle valve member (41) has the form of a hollow cylinder with an open end towards the circulator (17) and at its other end a transverse wall (42) having an aperture (43) aligned with the aperture (13) in the web (12) in the barrel (10).

7. ·.      A pump as claimed in any one of the preceding claims characterised in that the barrel (10) and reservoir bottle (23) are made of transparent plastics material.

8.      A pump as claimed in any one of the preceding claims characterised in that the filter element (28) is a filter sock and the barrel (10) includes an open structure extending from the filter seating (25) to support the filter sock from the inside thereof against compression during liquid flow along the efflux path.

9.        A pump as claimed in any one of claims 1 to 7 characterised in that the filter element (28) is self-supporting in compression.

FIG. 1.

FIG. 2.

FIG. 3.

FIG. 4.

## European Patent Office

## EUROPEAN SEARCH REPORT

| | DOCUMENTS CONSIDERED TO BE RELEVANT | | EP 85309476.1 |
|---|---|---|---|

| Category | Citation of document with indication, where appropriate, of relevant passages | Relevant to claim | CLASSIFICATION OF THE APPLICATION (Int Cl.4) |
|---|---|---|---|
| D,A | US - A - 3 892 226 (I. CH. ROSEN)<br>* Totality; especially fig. 3, 6; column 4, line 6 - column 5, line 28 * | 1 | A 61 M 1/00<br>A 61 M 7/00 |
| D,A | US - A - 1 925 230 (H. L. BUCKHOUT)<br>* Totality * | 1 | |
| D,A | US - A - 4 282 873 (R.A.ROTH)<br>* Totality; especially fig. 1; column 6, line 25 - column 7, line 9 * | 1 | |
| D,A | GB - A - 2 136 690 (G.TH. WATTS)<br>* Totality * | 1 | TECHNICAL FIELDS SEARCHED (Int. Cl.4) |
| A | US - A - 4 029 095 (J.B.PENA)<br>* Totality; especially fig. 2; column 4, line 42 - column 5, line 34 * | 1 | A 61 M 1/00<br>A 61 M 7/00 |

The present search report has been drawn up for all claims

| Place of search | Date of completion of the search | Examiner |
|---|---|---|
| VIENNA | 13-05-1986 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82